# EUROPEAN PATENT APPLICATION

(11) **EP 4 600 257 A1**
(43) Date of publication of application: **13.08.2025**
(21) Application number: 23880247.4
(22) Date of filing: 19.10.2023
(51) Int. Cl.: C07K 14/34, C12N 15/77, C12P 13/08

(54) **MUTANT RIBONUCLEASE ACTIVITY REGULATING PROTEIN AND METHOD FOR PRODUCING L-VALINE USING SAME**

(30) Priority: 19.10.2022 KR 20220135102
(71) Applicant: CJ CheilJedang Corporation, Seoul 04560 (KR)
(72) Inventor: KIM, Seon Hye, Seoul 04560 (KR); OH, Haena, Seoul 04560 (KR); YUN, Hyojin, Seoul 04560 (KR); LEE, Hyein, Seoul 04560 (KR)
(74) Representative: Meissner Bolte Partnerschaft mbB
(86) International application number: PCT/KR2023/016215
(87) International publication number: WO 2024/085671

(57) **Abstract**

The present disclosure relates to a ribonuclease activity regulator protein variant, and a method of producing L-valine using the same.

## Description

### [Technical Field]

The present disclosure relates to a ribonuclease activity regulator protein variant, and a method of producing L-valine using the same.

### [Background Art]

L-amino acids are the basic structural blocks of proteins, and are used as important materials such as pharmaceutical raw materials and food additives, animal feeds, nutrients, pesticides, bactericides, etc.

In particular, branched-chain amino acids (BCAAs) collectively refer to L-valine, L-leucine, and L-isoleucine, which are essential amino acids, which have an antioxidant effect and an effect on promoting protein synthesis in muscle cells.

Production of branched-chain amino acids using microorganisms is mainly carried out through microorganisms of the genus *Corynebacterium.*

For example, in the case of L-valine, target-specific approaches have been mainly used, such as a method of increasing expression of a gene encoding an enzyme involved in its biosynthesis or a method of removing a gene unnecessary for the biosynthesis (US 8465962 B2, KR 10-2153534 B1). In addition, a method of producing L-valine through feedback inhibition has been studied (US 10457919 B2).

### [Disclosure]

### [Technical Problem]

The present inventors found that when a ribonuclease activity regulator protein variant is introduced into a microorganism, its L-valine-producing ability is increased, as compared to a microorganism comprising a wild-type protein, thereby completing the present disclosure.

### [Technical Solution]

The present disclosure provides a ribonuclease activity regulator protein variant, in which an amino acid corresponding to position 93 in an amino acid sequence of SEQ ID NO: 1 is substituted with a different amino acid.

The present disclosure provides a polynucleotide encoding the ribonuclease activity regulator protein variant of the present disclosure.

The present disclosure provides a microorganism comprising the ribonuclease activity regulator protein variant of the present disclosure; or the polynucleotide encoding the same.

The present disclosure provides a method of producing L-valine, the method comprising the step of culturing, in a medium, the microorganism comprising the ribonuclease activity regulator protein variant of the present disclosure; or the polynucleotide encoding the same.

### [Advantageous Effects]

When a microorganism producing L-valine is cultured using a ribonuclease activity regulator protein variant of the present disclosure, it is possible to produce L-valine with high yields, as compared to a microorganism having the existing wild-type ribonuclease activity regulator protein.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a ribonuclease activity regulator protein variant, in which an amino acid corresponding to position 93 in an amino acid sequence of SEQ ID NO: 1 is substituted with a different amino acid.

As used herein, the term "ribonuclease activity regulator protein variant" refers to a variant comprising a substitution of an amino acid corresponding to position 93 from the N-terminus of SEQ ID NO: 1 with a different amino acid in any polypeptide having the function of regulating ribonuclease activity or the ribonuclease activity regulator protein. The "variant ribonuclease activity regulator protein" may also be referred to as "ribonuclease activity regulator protein variant", "RraA variant", "variant RraA", "rraA variant", "variant rraA", etc.

The protein that is the target of mutation introduction in the present disclosure may be a protein with an activity that regulates ribonuclease activity. Specifically, the protein may comprise the amino acid sequence of SEQ ID NO: 1 and may have an activity that regulates ribonuclease activity, but is not limited thereto. The addition of a meaningless sequence upstream or downstream of the amino acid sequence of SEQ ID NO: 1, a naturally occurring mutation, or a silent mutation thereof is not excluded, and as long as a protein has an activity identical or corresponding to the activity of the protein comprising the amino acid sequence of SEQ ID NO: 1, it may belong to the protein that is the target of mutation introduction of the present disclosure. For example, the protein that is the target of mutation introduction of the present disclosure may be a protein consisting of the amino acid sequence of SEQ ID NO: 1 or an amino acid sequence having 80%, 85%, 90%, 95%, 96%, 97%, 98%, or 99% or more homology or identity thereto. It is also apparent that a protein having an amino acid sequence having deletion, modification, substitution, or addition of some sequence also falls within the scope of the protein that is the target of mutation introduction of the present disclosure as long as the amino acid sequence has such homology or identity and exhibits efficacy corresponding to that of the protein.

As used herein, the term "ribonuclease activity regulator protein" refers to a protein that regulates ribonuclease activity by binding to endonuclease RNase E and inhibiting RNA processing, and may be used interchangeably with "ribonuclease E activity regulator or RraA". The amino acid sequence of RraA may be obtained from known databases such as NCBI's Genbank, etc.

For example, the RraA protein of the present disclosure may be derived from a microorganism, specifically, a prokaryotic microorganism or a eukaryotic microorganism, and more specifically, a microorganism of the genus *Corynebacterium,* etc., but is not limited thereto.

For another example, the RraA protein may be WP_003858525.1 derived from a microorganism of the genus *Corynebacterium,* and it is apparent that the RraA protein comprises proteins having activity that regulates ribonuclease activity, which are derived from various sources.

In the amino acid sequence before modification of the RraA protein, which is the mutation target in the present disclosure, the amino acid before modification, corresponding to position 93 of SEQ ID NO: 1, may be histidine (H).

Regarding the ribonuclease activity regulator protein variant of the present disclosure, the amino acid corresponding to position 93 in the amino acid sequence of SEQ ID NO: 1 may be substituted with an amino acid different from the amino acid before substitution. Alternatively, the ribonuclease activity regulator protein variant may be a ribonuclease activity regulator protein variant having a substitution with an amino acid different from the amino acid before substitution, which is a polar or hydrophilic amino acid among amino acids having an uncharged side chain (uncharged amino acids), but is not limited thereto.

For example, with regard to the ribonuclease activity regulator protein variant, the amino acid corresponding to position 93 in the amino acid sequence of SEQ ID NO: 1 may be substituted with an amino acid selected from the group consisting of tyrosine, arginine, lysine, aspartic acid, asparagine, alanine, valine, leucine, isoleucine, methionine, phenylalanine, tryptophan, proline, serine, threonine, cysteine, glycine, glutamic acid, and glutamine.

For another example, with regard to the ribonuclease activity regulator protein variant, the amino acid corresponding to position 93 in the amino acid sequence of SEQ ID NO: 1 may be substituted with an amino acid selected from the group consisting of tyrosine, glycine, alanine, valine, leucine, isoleucine, methionine, phenylalanine, tryptophan, proline, serine, threonine, cysteine, tyrosine, asparagine, and glutamine, for example, tyrosine.

For still another example, the ribonuclease activity regulator protein variant of the present disclosure may have, comprise, or consist of an amino acid sequence represented by SEQ ID NO: 3 or an amino acid sequence having at least 70%, 75%, 80%, 85%, 90%, 95%, 96%, 97%, 98%, 99%, 99.1%, 99.2%, 99.3%, or 99.4% or more homology or identity thereto, or may essentially consist of the amino acid sequence.

The ribonuclease activity regulator protein variant of the present disclosure may comprise an amino acid sequence, in which the amino acid corresponding to position 93 in the amino acid sequence of SEQ ID NO: 1 is an amino acid other than histidine, e.g., tyrosine, the amino acid sequence having at least 70%, 75%, 80%, 85%, 90%, 95%, 96%, 97%, 98%, 99%, 99.1%, 99.2%, 99.3%, or 99.4% or more homology or identity to the amino acid sequence represented by SEQ ID NO: 1. It is also apparent that a ribonuclease activity regulator protein variant with an amino acid sequence having deletion, modification, substitution, conservative substitution, or addition of some sequence also falls within the scope of the present disclosure as long as the amino acid sequence has such homology or identity and exhibits efficacy corresponding to that of the ribonuclease activity regulator protein variant of the present disclosure.

For example, the variant may comprise those having addition or deletion of a sequence that do not alter the function of the ribonuclease activity regulator protein variant of the present disclosure, at the N-terminus, C-terminus, and/or inside of the amino acid sequence, or a naturally occurring mutation, a silent mutation, or a conservative substitution.

The "conservative substitution" refers to substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, conservative substitution may hardly affect or not affect activity of proteins or polypeptides.

For example, among the amino acids having an electrically charged side chain (electrically charged amino acids), positively charged (basic) amino acids comprise arginine, lysine, and histidine, and negatively charged (acidic) amino acids comprise glutamic acid and aspartic acid; and amino acids having an uncharged side chain comprise glycine, alanine, valine, leucine, isoleucine, methionine, phenylalanine, tryptophan, proline, serine, threonine, cysteine, tyrosine, asparagine, and glutamine.

As used herein, the term "protein variant" or "variant" refers to a polypeptide which has an amino acid sequence different from that of the variant before modification by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased, as compared to that of the polypeptide before variation. Further, some variants may comprise variants in which one or more portions such as an N-terminal leader sequence or a transmembrane domain have been removed. Other variants may comprise variants in which a portion of the N- and/or C-terminus has been removed from the mature protein. The term "protein variant" may be used interchangeably with terms such as modification, modified polypeptide, modified protein, mutant, mutein, divergent, etc., and is not limited thereto as long as it is a term used with the meaning of variation. With respect to the objects of the present disclosure, the variant may be a polypeptide in which the amino acid corresponding to position 93 in the amino acid sequence of SEQ ID NO: 1 may be substituted with serine. For example, the variant may be a polypeptide comprising the amino acid sequence represented by SEQ ID NO: 3, but is not limited thereto.

Further, the variant may comprise deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. Further, the variant may be conjugated with other sequences or linkers to be identified, purified, or synthesized.

As used herein, the term 'homology' or 'identity' means the degree of similarity between two given amino acid sequences or base sequences and may be expressed as a percentage. The terms 'homology and identity' may often be used interchangeably.

The sequence homology or identity of a conserved polynucleotide or polypeptide is determined by standard alignment algorithms, and the default gap penalty established by a program to be used may be used together. Substantially, homologous or identical sequences are generally capable of being hybridized with the entirety or a part of the sequence under moderately or highly stringent conditions. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387 (1984)), BLASTP, BLASTN, FASTA (Atschul, [S.] [F.,] [ETAL, J MOLEC BIOL 215]: 403 (1990); Guide to Huge Computers, Martin J. Bishop, [ED.,] Academic Press, San Diego,1994, and [CARILLO et al/.](1988) SIAM J Applied Math 48: 1073). For example, the homology, similarity, or identity may be determined using BLAST or ClustalW of the National Center for Biotechnology Information.

The homology, similarity, or identity of polynucleotides or polypeptides may be, for example, determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of symbols in the shorter of the two sequences. Default parameters for the GAP program may comprise (1) a binary comparison matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

For one example of the present disclosure, the ribonuclease activity regulator protein variant of the present disclosure may have an activity to increase the L-valine-producing ability, as compared to the wild-type polypeptide having the activity regulating the ribonuclease activity.

As used herein, the term "corresponding to" refers to an amino acid residue at the position recited in a polypeptide, or an amino acid residue which is similar, identical, or homologous to the residue recited in a polypeptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions or deletions occur, compared to a query sequence (also referred to as a "reference sequence").

In such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), etc. may be used, but is not limited thereto, and a sequence alignment program, a pairwise sequence comparison algorithm, etc., which known in the art, may be appropriately used.

Another aspect of the present disclosure provides a polynucleotide encoding the ribonuclease activity regulator protein variant of the present disclosure.

The RraA protein of the present disclosure may be encoded by *rraA* gene.

For example, the *rraA* gene may be a polynucleotide encoding WP_003858525.1 derived from a microorganism of the genus *Corynebacterium,* but is not limited thereto. For another example, the *rraA* gene may be *rraA* gene derived from a microorganism of the genus *Corynebacterium,* but is not limited thereto, and it is apparent that the *rraA* gene comprises *rraA* genes encoding proteins having the RraA protein activity, which are derived from various sources.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the ribonuclease activity regulator protein variant.

The polynucleotide fragment encoding the ribonuclease activity regulator protein variant of the present disclosure may comprise a nucleotide sequence encoding the ribonuclease activity regulator protein variant in which the amino acid corresponding to position 93 in the amino acid sequence of SEQ ID NO: 1 is substituted with a different amino acid, or a nucleotide sequence in which the codon corresponding to positions 277 to 279 in the nucleotide sequence of SEQ ID NO: 2 is substituted with a codon encoding a different amino acid. For example, the polynucleotide of the present disclosure may comprise a nucleotide sequence encoding the amino acid sequence represented by SEQ ID NO: 3. For more specific example of the present disclosure, the polynucleotide of the present disclosure may have or comprise a sequence of SEQ ID NO: 4. Further, the polynucleotide of the present disclosure may consist of or essentially consist of the sequence of SEQ ID NO: 4.

In the polynucleotide of the present disclosure, various modifications may be made in the coding region as long as the amino acid sequence of the ribonuclease activity regulator protein variant of the present disclosure is not changed in consideration of codon degeneracy or codons preferred in organisms that are intended to express the ribonuclease activity regulator protein variant of the present disclosure. Specifically, the polynucleotide of the present disclosure may have or comprise a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 4, or may consist of or essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 4, but is not limited thereto. In this regard, in the sequence having the homology or identity, the codon encoding the amino acid corresponding to position 93 of SEQ ID NO: 1 may be any one of codons encoding an amino acid other than histidine, e.g., tyrosine.

Further, the polynucleotide of the present disclosure may comprise a probe that may be prepared from a known gene sequence, for example, any sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof may comprise a condition in which polynucleotides having higher homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically 68°C, 0.1XSSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relationship between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

Still another aspect of the present disclosure provides a vector comprising the polynucleotide of the present disclosure.

The vector may be an expression vector for expressing the polynucleotide in a host cell, but is not limited thereto.

The vector of the present disclosure may comprise a DNA construct comprising the nucleotide sequence of the polynucleotide encoding the target polypeptide that is operably linked to a suitable expression regulatory region (or expression control sequence) so that the target polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then may replicate or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a target polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting cells transformed with vectors, i.e., for confirming the insertion of a target nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a target polypeptide. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the target ribonuclease activity regulator protein variant of the present disclosure.

Still another aspect of the present disclosure provides a microorganism comprising the ribonuclease activity regulator protein variant of the present disclosure or the polynucleotide of the present disclosure.

The strain of the present disclosure may comprise the ribonuclease activity regulator protein variant of the present disclosure, the polynucleotide encoding the polypeptide, or the vector comprising the polynucleotide of the present disclosure.

As used herein, the term "microorganism (or strain)" comprises all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, etc., and it may be a microorganism comprising genetic modification for production of a target polypeptide, protein, or product.

The strain of the present disclosure may be a strain naturally having L-valine-producing ability or a microorganism prepared by providing the L-valine-producing ability for a strain without the L-valine-producing ability. For example, it may be a microorganism in which the L-valine-producing ability is increased by introducing the ribonuclease activity regulator protein variant of the present disclosure or the polynucleotide encoding the same, but is not limited thereto.

The strain of the present disclosure may be a microorganism in which the L-valine-producing ability is increased, as compared to a parent strain or wild-type strain of the genus *Corynebacterium* without the variant of the present disclosure. The microorganism may be a microorganism in which the L-valine-producing ability is improved by introducing the variant of the present disclosure.

For example, the ribonuclease activity regulator protein-unmodified microorganism, which is a target strain for comparing whether or not the L-valine-producing ability is increased, may be an L-valine-producing strain *Corynebacterium glutamicum* KCCM11201P (CA08-0072, US 8465962 B2), an ilvN(A42V) mutation-introduced wild-type *Corynebacterium glutamicum* ATCC14067 (Korean Patent No. 10-1947945), or a wild-type *Corynebacterium glutamicum*ATCC13869 (Korean Patent No. 10-1947945), but is not limited thereto.

For example, the recombinant strain with the increased production ability may have an L-valine-producing ability of about 1% or more, specifically, about 2% or more, about 3% or more, about 4% or more, about 5% or more, about 6% or more, about 7% or more, about 8% or more, about 9% or more, about 10% or more, about 11% or more, about 12% or more, about 13% or more, or about 14% or more (the upper limit is not particularly limited, but may be, for example, about 200% or less, about 150% or less, about 100% or less, about 50% or less, about 40% or less, about 30% or less, about 20% or less, or about 15% or less), as compared to the parent strain before modification or unmodified microorganism, but the increased amount is not limited thereto as long as the production ability has an increased amount of a + value, as compared to the production ability of the parent strain before modification, the unmodified microorganism, or the ribonuclease activity regulator protein-unmodified microorganism. In another example, the recombinant strain having the increased L-valine-producing ability may have an L-valine-producing ability of about 1.01 times or more, about 1.02 times or more, about 1.03 times or more, about 1.04 times or more, about 1.05 times or more, about 1.06 times or more, about 1.07 times or more, about 1.08 times or more, about 1.09 times or more, about 1.10 times or more, about 1.11 times or more, about 1.12 times or more, about 1.13 times or more, or about 1.14 times or more (the upper limit is not particularly limited, but may be, for example, about 10 times or less, about 5 times or less, about 3 times or less, or about 2 times or less), as compared to the parent strain before modification, the unmodified microorganism, or the ribonuclease activity regulator protein-unmodified microorganism, but is not limited thereto.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. Further, as used herein, the term "ribonuclease activity regulator protein-unmodified microorganism" may refer to a strain into which the ribonuclease activity regulator protein variant described herein is not introduced or has not yet been introduced. The ribonuclease activity regulator protein-unmodified microorganism of the present disclosure does not exclude a strain comprising other protein or other gene modification than modification of the ribonuclease activity regulator protein or the polynucleotide encoding the same.

As used herein, the term "unmodified microorganism" may be used interchangeably with "strain before being modified", "microorganism before being modified", "unvaried strain", "unmodified strain", "unvaried microorganism", or "reference microorganism".

The microorganism of the present disclosure may be a microorganism comprising the ribonuclease activity regulator protein variant or the polynucleotide encoding the same; or a microorganism which is genetically modified to comprise the ribonuclease activity regulator protein variant or the polynucleotide encoding the same (e.g., recombinant microorganism), but is not limited thereto. The "endogenous activity" means the activity of a specific polypeptide originally possessed by the parent strain prior to the change in the trait or by a wild-type or unmodified microorganism before the trait is changed by genetic variation due to natural or artificial factors. This may be used interchangeably with "activity before modification".

For still another example of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium glutamicum,* but is not limited thereto.

For still another example of the present disclosure, the recombinant microorganism of the present disclosure may be a microorganism in which the L-valine-producing ability is enhanced by additionally enhancing the activity of some of the proteins involved in the L-valine biosynthesis pathway, or by additionally weakening the activity of some of the proteins involved in the L-valine degradation pathway (US 8465962 B2, KR 10-2153534 B1).

For still another example of the present disclosure, the recombinant microorganism of the present disclosure may be a microorganism in which the L-valine-producing ability is enhanced by additionally inducing feedback inhibition (US 10457919 B2).

For still another example of the present disclosure, the recombinant microorganism of the present disclosure may be a microorganism in which the L-valine-producing ability is enhanced by enhancing the activity of acetolactate synthase isozyme 1 small subunit (IlvN) protein.

In any one embodiment of the above-described embodiments, the recombinant microorganism of the present disclosure may be a microorganism in which the L-valine-producing ability is enhanced by enhancing the activity of the IlvN protein due to introduction of one mutation [ilvN(A42V); Biotechnology and Bioprocess Engineering, June 2014, Volume 19, Issue 3, pp 456-467] into the IlvN protein.

The "acetolactate synthase isozyme 1 small subunit (IlvN) protein" of the present disclosure has an activity to catalyze the conversion of two pyruvate molecules to acetolactate in a first common step of the biosynthetic pathway of branched-chain amino acids (BCAAs), comprising L-valine, L-leucine, and L-isoleucine, and the microorganism mutated to enhance the activity of the IlvN protein has a characteristic of enhancing the L-valine biosynthetic pathway, and thus may be usefully applied to the production of L-valine. For example, the microorganism mutated to enhance the activity of the IlvN protein may be a microorganism into which ilvN (A42V) has been introduced.

The ilvN(A42V) of the present disclosure may be a protein having or comprising an amino acid sequence represented by SEQ ID NO: 13, or a protein consisting of or essentially consisting of the amino acid sequence represented by SEQ ID NO: 13, but is not limited thereto. Further, the ilvN(A42V) of the present disclosure may have or comprise an amino acid sequence having at least 70%, 80%, 90%, 95% or 99% or more homology or identity to the amino acid sequence represented by SEQ ID NO: 13, as long as it exhibits the activity of ilvN(A42V). In addition, the ilvN(A42V) of the present disclosure may consist of or essentially consist of an amino acid sequence having at least 70%, 80%, 90%, 95% or 99% or more homology or identity to the amino acid sequence represented by SEQ ID NO: 13, but is not limited thereto. Further, a polynucleotide encoding the ilvN(A42V) may have or comprise a nucleotide sequence encoding the amino acid sequence represented by SEQ ID NO: 13. In addition, the polynucleotide encoding the ilvN(A42V) may consist of or essentially consist of the nucleotide sequence encoding the amino acid sequence represented by SEQ ID NO: 13. In the polynucleotide encoding the ilvN(A42V) of the present disclosure, various modifications may be made in the coding region as long as the amino acid sequence of the ilvN(A42V) protein is not changed in consideration of codon degeneracy or codons preferred in organisms where the ilvN(A42V) protein is intended to express. The polynucleotide encoding the ilvN(A42V) of the present disclosure may have or comprise a nucleotide sequence having at least 70%, 80%, 90%, 95% or 99% or more, and less than 100% homology or identity to the nucleotide sequence of SEQ ID NO: 14. In addition, the polynucleotide encoding the ilvN(A42V) of the present disclosure may consist of or essentially consist of a nucleotide sequence having at least 70%, 80%, 90%, 95% or 99% or more, and less than 100% homology or identity to the nucleotide sequence of SEQ ID NO: 14, but is not limited thereto.

In one embodiment, the ilvN(A42V)-introduced microorganism may be *Corynebacterium glutamicum* CJ7V or *Corynebacterium glutamicum* CJ8V, but is not limited thereto. In addition to the above description, descriptions disclosed in Korean Patent No. 10-1947945, etc. regarding such L-valine-producing microorganisms may be used as reference for the present disclosure, but is not limited thereto.

As used herein, the term "weakening" of activity of a polypeptide (e.g., comprising a protein identified by the name of each enzyme) is a concept comprising both cases where the activity is decreased, as compared to the endogenous activity, or the activity is absent. The weakening may be used interchangeably with terms such as inactivation, deficiency, down-regulation, decrease, reduce, attenuation, etc.

The weakening may also comprise a case where the activity of the polypeptide itself is decreased or eliminated due to variation of the polynucleotide encoding the polypeptide, etc., as compared to the activity of the polypeptide originally possessed by the microorganism, a case where the overall polypeptide activity level and/or concentration (expression level) in the cell is low due to inhibition of the expression of the gene of the polynucleotide encoding the polypeptide or by inhibition of translation into the polypeptide, as compared to that of the natural strain, a case where the polynucleotide is not expressed at all, and/or a case where the polypeptide activity is absent even when the polynucleotide is expressed. The fact that the activity of the polypeptide is "inactivated, deficient, decreased, down-regulated, reduced, or attenuated" as compared to the endogenous activity means that the activity of the polypeptide is lowered, as compared to the activity of a specific polypeptide originally possessed by the parent strain before change of the trait or the unmodified microorganism.

Such weakening of the activity of a polypeptide may be performed by any method known in the art, but the method is not limited thereto, and the weakening may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide activity of the present disclosure may be:
1) deletion of the entirety or a part of the gene encoding the polypeptide;
2) modification of an expression regulatory region (or expression regulatory sequence) to decrease expression of the gene encoding the polypeptide;
3) modification of an amino acid sequence constituting the polypeptide to eliminate or weaken the activity of the polypeptide (e.g., deletion/substitution/addition of one or more amino acids in the amino acid sequence);
4) modification of a gene sequence encoding the polypeptide to eliminate or weaken the activity of the polypeptide (e.g., deletion/substitution/addition of one or more nucleic acid bases in a nucleic acid sequence of the polypeptide gene to encode the polypeptide that has been modified to eliminate or weaken the activity of the polypeptide);
5) modification of a start codon of a gene transcript encoding the polypeptide or a nucleotide sequence encoding a 5'-UTR region;
6) introduction of an antisense oligonucleotide (e.g., antisense RNA) that complementarily binds to the transcript of the gene encoding the polypeptide;
7) addition of a sequence complementary to a Shine-Dalgarno sequence in front of the Shine-Dalgarno sequence of the gene encoding the polypeptide in order to form a secondary structure to which ribosome cannot be attached;
8) addition of a promoter to be transcribed in the opposite direction to the 3' end of the open reading frame (ORF) of the gene sequence encoding the polypeptide (reverse transcription engineering, RTE); or
9) a combination of two or more selected from 1) to 8), but is not particularly limited thereto.

For example,
1) the deletion of a part or the entirety of the gene encoding the polypeptide may be removal of the entire polynucleotide encoding the endogenous target polypeptide in the chromosome, replacement with a polynucleotide in which some nucleotides are deleted, or replacement with a marker gene.

Further, 2) the modification of the expression regulatory region (or expression regulatory sequence) may be deletion, insertion, non-conservative or conservative substitution, or occurrence of variation in the expression regulatory region (or expression regulatory sequence) due to a combination thereof, or replacement with a sequence exhibiting weaker activity. The expression regulatory region comprises a promoter, an operator sequence, a sequence encoding a ribosome binding site, and a sequence regulating the termination of transcription and translation, but is not limited thereto.

3) and 4) The modification of the amino acid sequence or polynucleotide sequence may be deletion, insertion, or non-conservative or conservative substitution of the amino acid sequence of the polypeptide or the polynucleotide sequence encoding the polypeptide or occurrence of variation in the sequence due to a combination thereof or replacement with an amino acid sequence or a polynucleotide sequence modified to exhibit weaker activity or an amino acid sequence or a polynucleotide sequence modified to be inactive so that the activity of the polypeptide is weakened, but is not limited thereto. For example, expression of the gene may be inhibited or weakened by introducing variation into the polynucleotide sequence and forming a stop codon, but is not limited thereto.

5) The modification of a start codon of a gene transcript encoding the polypeptide or a nucleotide sequence encoding a 5'-UTR region may be, for example, substitution with a nucleotide sequence encoding another start codon having a lower polypeptide expression rate, as compared to an endogenous start codon, but is not limited thereto.

6) the introduction of an antisense oligonucleotide (e.g., antisense RNA) that complementarily binds to the transcript of the gene encoding the polypeptide, may refer to documents, for example, [Weintraub, H. et al., Antisense-RNA as a molecular tool for genetic analysis, Reviews - Trends in Genetics, Vol. 1(1) 1986].

7) The addition of a sequence complementary to a Shine-Dalgarno sequence in front of the Shine-Dalgarno sequence of the gene encoding the polypeptide in order to form a secondary structure to which ribosome cannot be attached may be to make mRNA translation impossible or to slow down the mRNA translation rate.

Further, 8) the addition of a promoter to be transcribed in the opposite direction to the 3' end of the open reading frame (ORF) of the gene sequence encoding the polypeptide (reverse transcription engineering, RTE) may be to weaken the activity by making an antisense nucleotide complementary to the transcript of the gene encoding the polypeptide.

As used herein, the term "enhancement" of activity of a polypeptide means that the activity of the polypeptide is increased compared to the endogenous activity. The enhancement may be used interchangeably with terms such as activation, up-regulation, overexpression, increase, etc. Here, activation, enhancement, up-regulation, overexpression, and increase may comprise both exhibiting activity that was not originally possessed and exhibiting improved activity, as compared to the endogenous activity or activity before modification. The fact that the activity of the polypeptide is "enhanced", "up-regulated", "overexpressed", or "increased", as compared to the endogenous activity, means that the activity of the polypeptide is improved, as compared to the activity and/or concentration (expression level) of a specific polypeptide originally possessed by a parent strain before the change in the trait or by an unmodified microorganism.

The enhancement may be achieved through the introduction of a foreign polypeptide or the enhancement of endogenous activity and/or concentration (expression level) of the polypeptide. The activity enhancement of the polypeptide may be confirmed by an increase in the degree of activity and the expression level of the corresponding polypeptide or in the amount of a product produced from the corresponding polypeptide.

For the activity enhancement of the polypeptide, various methods well known in the art may be applied, and the method is not limited as long as the activity of the target polypeptide may be enhanced, as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the activity enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a start codon of a gene transcript encoding the polypeptide or a nucleotide sequence encoding a 5'-UTR region;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the polypeptide;
7) codon optimization of a polynucleotide encoding the polypeptide;
8) analysis of the tertiary structure of the polypeptide to select the exposed site and to perform modification or chemical modification of the exposed site; or
9) a combination of two or more selected from 1) to 8), but is not particularly limited thereto.

More specifically:
1) The increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.
2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may comprise, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.

Examples of known strong promoters comprise CJ1 to CJ7 promoters (US Patent No. 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US Patent No. 10584338 B2), an O2 promoter (US Patent No. 10273491 B2), a tkt promoter, an yccA promoter, etc., but is not limited thereto.

3) The modification of a start codon of a gene transcript encoding the polypeptide or a nucleotide sequence encoding a 5'-UTR region may be, for example, substitution with a nucleotide sequence encoding another start codon having a higher polypeptide expression rate as compared to an endogenous start codon, but is not limited thereto.

4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further comprise a selection marker for the confirmation of chromosome insertion.

6) The introduction of a foreign polynucleotide exhibiting the activity of the polypeptide may be introduction of a foreign polynucleotide encoding a polypeptide exhibiting activity identical or similar to that of the polypeptide into a host cell. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.

7) The codon optimization of the polynucleotide encoding the polypeptide may be codon optimization of an endogenous polynucleotide so as to increase transcription or translation in a host cell, or codon optimization of a foreign polynucleotide so as to perform optimized transcription and translation in a host cell.

8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type or a microbial strain before being modified, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may comprise a method of using a DNA recombination technology. For example, by introducing a nucleotide sequence or vector comprising a nucleotide sequence homologous to the target gene into the microorganism to cause homologous recombination, a part or the entirety of the gene may be deleted. The nucleotide sequence or vector to be introduced may comprise a dominant selection marker, but is not limited thereto.

In the microorganism of the present disclosure, the ribonuclease activity regulator protein variant, the polynucleotide, and the L-valine, etc. are as described in other aspects.

Still another aspect of the present disclosure provides a method of producing L-valine, the method comprising the step of culturing, in a medium, the microorganism comprising the ribonuclease activity regulator protein variant of the present disclosure or the polynucleotide of the present disclosure.

The method of producing L-valine of the present disclosure may comprise the step of culturing, in a medium, a microorganism comprising the ribonuclease activity regulator protein variant of the present disclosure, the polynucleotide of the present disclosure, or the vector of the present disclosure.

As used herein, the term "culturing" refers to growing the microorganism of the present disclosure in appropriately adjusted environment conditions. The culture procedure of the present disclosure may be performed according to appropriate media or culture conditions known in the art. Such culturing process may be easily adjusted and used by a person skilled in the art according to the selected microorganism. Specifically, the culturing may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the microorganism of the present disclosure, any medium that is commonly used for culturing microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, while controlling the temperature, pH, etc.

Specifically, a culture medium for microorganisms of the genus *Corynebacterium* may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, pH of the medium may be adjusted by adding compounds, such as ammonium hydroxide, potassium hydroxide, ammonia, phosphoric acid, and sulfuric acid, to the medium in an appropriate manner during culturing of the microorganism of the present disclosure. In addition, an anti-foaming agent, such as a fatty acid polyglycol ester, may be added to suppress foam formation during culturing. In addition, oxygen or oxygen-comprising gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culturing of the present disclosure, the culture temperature may be maintained at 20°C to 45°C, specifically 25°C to 40°C, and the culturing may be performed for about 10 hours to 160 hours, but is not limited thereto.

The L-valine produced by culturing of the present disclosure may be released into the medium or may remain in cells.

The method of producing L-valine of the present disclosure may further comprise the steps of preparing the microorganism of the present disclosure, preparing a medium for culturing the microorganism, or a combination of these steps (regardless of the order, in any order), for example, before the culturing step.

The method of producing L-valine of the present disclosure may further comprise the step of recovering the L-valine from the medium resulting from the culturing (medium in which culturing has been performed) or the *Corynebacterium glutamicum* strain. The recovering step may be further comprised after the culturing step.

The recovering may be collecting a desired L-valine by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and the desired L-valine may be recovered from the medium or microorganism by using an appropriate method known in the art.

In addition, the method of producing L-valine of the present disclosure may further comprise a purification step. The purification may be performed by using an appropriate method known in the art. In an exemplary embodiment, when the method of producing L-valine of the present disclosure comprises both the recovering step and the purification step, the recovering step and the purification step may be performed continuously or discontinuously regardless of the order, or may be performed simultaneously or integrated into one step, but is not limited thereto.

In the method of the present disclosure, the ribonuclease activity regulator protein variant, the polynucleotide, the vector, and the strain, etc. are as described in other aspects.

Still another aspect of the present disclosure provides a composition for producing L-valine, the composition comprising the ribonuclease activity regulator protein variant of the present disclosure, the polynucleotide encoding the same, the vector comprising the polynucleotide, or the microorganism comprising the polynucleotide of the present disclosure; the medium in which the microorganism is cultured; or a combination of two or more thereof.

The composition of the present disclosure may further comprise any appropriate excipient that is commonly used in the composition for producing L-amino acids, and such excipients may comprise, for example, a preserving agent, a wetting agent, a dispersing agent, a suspending agent, a buffer, a stabilizing agent, an isotonic agent, etc., but are not limited thereto.

Still another aspect of the present disclosure provides use of the ribonuclease activity regulator protein variant of the present disclosure, the polynucleotide encoding the same, the vector comprising the polynucleotide, or the microorganism comprising the polynucleotide of the present disclosure in producing L-valine.

The ribonuclease activity regulator protein, the ribonuclease activity regulator protein variant, the polynucleotide, the vector, the strain, the medium, and the L-valine, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Preparation of mutation-induced strain

### Example 1-1. Mutagenesis

*Corynebacterium glutamicum* strain KCCM11201P (CA08-0072, US 8465962 B2), which is an L-valine-producing strain, was spread on a nutrient medium containing agar, and cultured at 30°C for 36 hours. Hundreds of colonies obtained through the culture were irradiated with UV at room temperature to induce random mutations in the genome of the strain.

### Example 1-2. Evaluation of L-valine-producing ability of mutation-induced strain

A fermentation titer test was conducted to select a strain, in which L-valine-producing ability was increased as compared to the parent strain KCCM11201P, from the strains in which random mutations were induced in Example 1-1. After subculturing each colony in a nutrient medium, each strain was inoculated into a 250 ml corner-baffled flask containing 25 ml of a production medium, and cultured at 30°C for 72 hours with shaking at 200 rpm. Thereafter, the concentrations of L-valine were evaluated using HPLC, and are shown in Table 1 below.

### <Nutrient medium>

10 g of glucose, 5 g of beef extract, 10 g of polypeptone, 2.5 g of sodium chloride, 5 g of yeast extract, 20 g of agar, 2 g of urea, pH 7.2 (based on 1 liter of distilled water)

### <Production medium>

100 g of glucose, 40 g of ammonium sulfate, 2.5 g of soy protein, 5 g of corn steep solids, 3 g of urea, 1 g of potassium phosphate dibasic, 0.5 g of magnesium sulfate heptahydrate, 100 µg of biotin, 1 mg of thiamine-HCl, 2 mg of calcium-pantothenic acid, 3 mg of nicotinamide, 30 g of calcium carbonate, pH 7.0 (based on 1 liter of distilled water), pH 7.0

**[Table 1]**

| | Name of strain | L-valine concentration (g/L) |
|---|---|---|
| Control group | KCCM11201P | 2.9 |
| Experimental group | C1 | 1.9 |
| | C2 | 2.8 |
| | C3 | 2.3 |
| | C4 | 2.1 |
| | C5 | 2.6 |
| | C6 | 1.8 |
| | C7 | 2.0 |
| | C8 | 3.2 |
| | C9 | 3.4 |
| | C10 | 4.1 |
| | C11 | 3.7 |
| | C12 | 2.1 |
| | C13 | 2.8 |
| | C14 | 1.6 |
| | C15 | 3.9 |

Referring to Table 1, C10 strain that showed the greatest increase in the L-valine-producing ability, as compared to the parent strain KCCM11201P, was selected.

### Example 2: Identification of mutation through gene sequencing

The major genes of the C10 strain selected in Example 1-2 were sequenced and compared with those of the parent strain KCCM11201P and the wild-type *Corynebacterium glutamicum* ATCC14067 strain. As a result, no mutations were found in the genes of the biosynthetic pathway, which are directly related to the L-valine-producing ability.

Accordingly, as a result of examining the presence or absence of a mutation through Next-Generation Sequencing (NGS) analysis, a mutation was found at a specific site in the open reading frame (ORF) region of the *rraA* gene encoding ribonuclease activity regulator protein (RraA). Specifically, it was found that, as compared to the parent strain KCCM11201P, the C10 strain had RraA (H93Y) (SEQ ID NO: 3) which is a variant in which one mutation was introduced into a base located 277 bp upstream from the start codon of the rraA gene, resulting in a change from the existing nucleotide sequence, CAT (SEQ ID NO: 2), to TAT (SEQ ID NO: 4), and histidine, which is an amino acid residue at position 93 from the N-terminus of the RraA protein, was substituted with tyrosine.

### Example 3: Construction of variant-expressing vector

To construct a vector introducing the H93Y mutation into the *rraA* gene, genomic DNA of C10 strain selected in Example 1-2 was extracted using a G-spin Total DNA Extraction Mini Kit (Cat. No 17045, Intron) according to the manufacturer's protocol, and PCR was performed using the genomic DNA as a template and a primer pair of SEQ ID NO: 5 and SEQ ID NO: 6. PCR conditions were as follows: denaturation at 94°C for 5 minutes; 25 cycles of denaturation at 94°C for 30 seconds, annealing at 55°C for 30 seconds, and polymerization at 72°C for 150 seconds; followed by polymerization reaction at 72°C for 7 minutes. As a result, 507 bp of a PCR product (hereinafter, referred to as "mutation-introduced fragment 1") was obtained.

The mutation-introduced fragment 1 obtained above was ligated into a pDCM2 vector (Korean Patent Publication No. 10-2020-0136813), which had been treated with a restriction enzyme Smal, using an Infusion Cloning Kit (Takara Bio Inc.), and transformed into *E*. *coli* DH5α by electroporation (Appl. Microbiol.Biotechnol. (1999) 52:541-545) to induce homologous recombination on the chromosome (van der Rest et al., Appl Microbiol Biotechnol 52:541-545, 1999). The strain in which the vector was inserted onto the chromosome by recombination of the homologous sequence was selected on an LB medium containing 25 mg/L kanamycin. DNA was obtained from the selected *E*. *coli* transformants using a DNA-spin plasmid DNA purification kit (Intron) according to the manufacturer's protocol, and a pDCM2-rraA(H93Y) vector containing the mutation-introduced fragment 1 for the introduction of the H93Y mutation into the *rraA* gene was constructed.

The primer sequences used here are as shown in Table 2 below.

**[Table 2]**

| SEQ ID NO. | Sequence name | Sequence (5' -> 3') |
|---|---|---|
| 5 | C10 rraA F | CCCGGG GTGGGCATGACTCAAAGTGCT |
| 6 | C10 rraA R | CCCGGG TTACTGCTTAATTGGCGCCTC |

### Example 4: Evaluation of L-valine-producing ability of variant-expressing strain

### Example 4-1. Evaluation of L-valine-producing ability of variant-expressing Corynebacterium glutamicum KCCM11201P strain

In order to prepare an L-valine-producing strain in which the H93Y mutation was introduced into the *rraA* gene, the pDCM2-rraA(H93Y) vector constructed in Example 3 was transformed into an L-valine-producing strain KCCM11201P by homologous recombination on the chromosome. The strain in which the vector was inserted into the chromosome by recombination of the homologous sequence was selected on a medium containing 25 mg/L kanamycin. Thereafter, PCR was performed using a primer pair of SEQ ID NO: 5 and SEQ ID NO: 6 on the *Corynebacterium glutamicum* transformant, in which the secondary recombination was completed, to amplify the gene fragment, and the strain in which the H93Y mutation was introduced into the *rraA* gene was identified through gene sequencing. The recombinant strain was named *Corynebacterium glutamicum* KCCM11201P::rraA(H93Y).

The L-valine-producing abilities of the parent strain KCCM11201P and the KCCM11201P::rraA(H93Y) strain were evaluated in the same manner as in Example 1-2, and are shown in Table 3 below.

**[Table 3]**

| Strain | L-valine concentration (g/L) | | | |
|---|---|---|---|---|
| | Batch 1 | Batch 2 | Batch 3 | Mean |
| KCCM11201P | 2.7 | 2.8 | 2.8 | 2.8 |
| KCCM11201P::rraA(H93Y) | 3.1 | 3.0 | 3.1 | 3.1 |

As a result, it was confirmed that the L-valine-producing ability of the KCCM11201P::rraA(H93Y) strain increased by 11%, as compared to the parent strain KCCM11201P.

### Example 4-2. Evaluation of L-valine-producing ability of variant-expressing Corynebacterium glutamicum CJ7V strain

In order to determine whether the introduction of the H93Y mutation into the *rraA* gene also increases the L-valine-producing ability in other L-valine-producing *Corynebacterium glutamicum* strains, a strain with improved L-valine-producing ability was prepared by introducing one mutation [ilvN(A42V); Biotechnology and Bioprocess Engineering, June 2014, Volume 19, Issue 3, pp 456-467] into the acetolactate synthase isozyme 1 small subunit (IlvN) protein of the wild-type *Corynebacterium glutamicum* ATCC14067 (Korean Patent No. 10-1947945).

First, to construct a vector for introducing the A42V mutation into the *ilvN* gene, genomic DNA of the wild-type *Corynebacterium glutamicum* ATCC14067 strain was extracted using a G-spin Total DNA extraction mini kit according to the manufacturer's protocol. PCR was performed using the genomic DNA as a template and a primer pair of SEQ ID NO: 7 and SEQ ID NO: 8 and a primer pair of SEQ ID NO: 9 and SEQ ID NO: 10 to obtain gene fragments A and B, respectively. The PCR conditions were as follows: denaturation at 94°C for 5 minutes; 25 cycles of denaturation at 94°C for 30 seconds, annealing at 55°C for 30 seconds, and polymerization at 72°C for 60 seconds; and polymerization at 72°C for 7 minutes. As a result, 528 bp of a gene fragment A and 509 bp of a gene fragment B were obtained. Overlapping PCR was performed using the above-obtained gene fragments A and B as templates and a primer pair of SEQ ID NO: 6 and SEQ ID NO: 9. As a result, 1010 bp of a PCR product (hereinafter, referred to as "mutation-introduced fragment 2") was obtained.

The above-obtained mutation-introduced fragment 2 was treated with a restriction enzyme Smal, ligated with a pDCM2 vector that had been treated with the same restriction enzyme, and transformed into *E*. *coli* DH5α by electroporation to induce homologous recombination on the chromosome. The strain in which the vector was inserted into the chromosome by recombination of the homologous sequence was selected on an LB medium containing kanamycin. DNA was obtained from the selected *E*. *coli* transformant using a DNA-spin plasmid DNA purification kit according to the manufacturer's protocol, and a pDCM2-ilvN(A42V) vector containing the mutation-introduced fragment 2 for the introduction of the A42V mutation into the *ilvN* gene was constructed.

The primer sequences used here are as shown in Table 4 below.

**[Table 4]**

| SEQ ID NO. | Sequence name | Sequence (5' -> 3') |
|---|---|---|
| 7 | primer 1 | cggggatcccccgggAGGACGGTACTCAAATACT AAACTTC |
| 8 | primer 2 | TGCCGAGTGTTTCGGTCTTTACAGACACGA GGGACACG |
| 9 | primer 3 | TGTCTGTAAAGACCGAAACACTCGGCATCA A |
| 10 | primer 4 | cggggatcccccgggGACAACTACATTATTATTATA CCACA |

The pDCM2-ilvN(A42V) vector constructed above was transformed into the wild-type *Corynebacterium glutamicum* ATCC14067 by homologous recombination on the chromosome. The strain in which the vector was inserted into the chromosome by recombination of the homologous sequence was selected on a medium containing 25 mg/L kanamycin. Thereafter, PCR was performed using a primer pair of SEQ ID NO: 7 and SEQ ID NO: 10 on the *Corynebacterium glutamicum* transformant, in which the secondary recombination was completed, to amplify the gene fragment, and the strain in which the A42V mutation was introduced into the *ilvN* gene was identified through gene sequencing. The recombinant strain was named *Corynebacterium glutamicum* CJ7V.

Finally, the pDCM2-rraA(H93Y) vector was transformed into the *Corynebacterium glutamicum* CJ7V in the same manner as Example 4-1. The above recombinant strain was named *Corynebacterium glutamicum* CJ7V::rraA(H93Y).

The L-valine-producing abilities of the parent strain CJ7V and the CJ7V::rraA(H93Y) strain were evaluated in the same manner as in Example 1-2, and are shown in Table 5 below.

**[Table 5]**

| Strain | L-valine concentration (g/L) | | | |
|---|---|---|---|---|
| | Batch 1 | Batch 2 | Batch 3 | Mean |
| CJ7V | 2.1 | 2.2 | 2.2 | 2.2 |
| CJ7V::rraA(H93Y) | 2.5 | 2.5 | 2.6 | 2.6 |

As a result, it was confirmed that the L-valine-producing ability of the CJ7V::rraA(H93Y) strain increased by 14%, as compared to the parent strain CJ7V.

### Example 4-3. Evaluation of L-valine-producing ability of variant-expressing Corynebacterium glutamicum CJ8V strain

A strain with improved L-valine-producing ability was prepared by introducing one mutation [ilvN (A42V)] into the IlvN protein of the wild-type *Corynebacterium glutamicum* ATCC13869 (Korean Patent No. 10-1947945).

The pDCM2-ilvN(A42V) vector constructed in Example 4-2 was transformed into the wild-type *Corynebacterium glutamicum* ATCC13869 strain. The strain in which the vector was inserted into the chromosome by recombination of the homologous sequence was selected on a medium containing 25 mg/L kanamycin. Thereafter, PCR was performed using a primer pair of SEQ ID NO: 11 and SEQ ID NO: 12 on the *Corynebacterium glutamicum* transformant, in which the secondary recombination was completed, to amplify the gene fragment, and the strain in which the A42V mutation was introduced into the *ilvN* gene was identified through gene sequencing. The recombinant strain was named *Corynebacterium glutamicum* CJ8V.

The primer sequences used here are as shown in Table 6 below.

**[Table 6]**

| SEQ ID NO. | Sequence name | Sequence (5' -> 3') |
|---|---|---|
| 11 | primer 5 | CCGCGTCACCAAAGCGGA |
| 12 | primer 6 | TTAGATCTTGGCCGGAGCCA |

Finally, the pDCM2-rraA(H93Y) vector was transformed into the *Corynebacterium glutamicum* CJ8V in the same manner as in Example 4-1. The above recombinant strain was named *Corynebacterium glutamicum* CJ8V::rraA(H93Y).

The L-valine-producing abilities of the parent strain CJ8V and the CJ8V::rraA(H93Y) strain were evaluated in the same manner as in Example 1-2, and are shown in Table 7 below.

**[Table 7]**

| Strain | L-valine concentration (g/L) | | | |
|---|---|---|---|---|
| | Batch 1 | Batch 2 | Batch 3 | Mean |
| CJ8V | 1.9 | 1.8 | 1.9 | 1.9 |
| CJ8V::rraA(H93Y) | 2.1 | 2.2 | 2.1 | 2.1 |

As a result, it was confirmed that the L-valine-producing ability of the CJ8V::rraA(H93Y) strain increased by 10%, as compared to the parent strain CJ8V.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A ribonuclease activity regulator protein variant, wherein an amino acid corresponding to position 93 in an amino acid sequence of SEQ ID NO: 1 is substituted with a different amino acid.

2. The ribonuclease activity regulator protein variant of claim 1, wherein the different amino acid is an amino acid selected from the group consisting of tyrosine, glycine, alanine, valine, leucine, isoleucine, methionine, phenylalanine, tryptophan, proline, serine, threonine, cysteine, tyrosine, asparagine, and glutamine.

3. The ribonuclease activity regulator protein variant of claim 1, wherein the different amino acid is tyrosine.

4. The ribonuclease activity regulator protein variant of claim 1, wherein the ribonuclease activity regulator protein variant has an amino acid sequence of SEQ ID NO: 3 or an amino acid sequence having 70% or more homology thereto.

5. A polynucleotide encoding the ribonuclease activity regulator protein variant of any one of claims 1 to 4.

6. A microorganism comprising a ribonuclease activity regulator protein variant, wherein an amino acid corresponding to position 93 in an amino acid sequence of SEQ ID NO: 1 is substituted with a different amino acid; or a polynucleotide encoding the same.

7. The microorganism of claim 6, wherein the microorganism has the increased L-valine-producing ability, as compared to a microorganism of the genus *Corynebacterium* comprising a wild-type ribonuclease activity regulator protein having the amino acid sequence of SEQ ID NO: 1 or a polynucleotide encoding the same.

8. The microorganism of claim 6, wherein the microorganism is a microorganism of the genus *Corynebacterium.*

9. The microorganism of claim 8, wherein the microorganism of the genus *Corynebacterium* is *Corynebacterium glutamicum.*

10. A method of producing L-valine, the method comprising the step of culturing, in a medium, a microorganism comprising a ribonuclease activity regulator protein variant, wherein an amino acid corresponding to position 93 in an amino acid sequence of SEQ ID NO: 1 is substituted with a different amino acid; or a polynucleotide encoding the same.
